# EUROPÄISCHE PATENTANMELDUNG

(11) **EP 4 644 702 A1**
(43) Veröffentlichungstag der Anmeldung: **05.11.2025**
(21) Anmeldenummer: 25170528.1
(22) Anmeldetag: 15.04.2025
(51) Int. Cl.: F04D 13/06, F04D 29/048, F04D 29/44, F04D 29/041, F04D 29/18, F04D 29/22, A61M 60/104, A61M 60/122, A61M 60/232, A61M 60/422, A61M 60/804, A61M 60/822

(54) **PUMPENEINHEIT FÜR EINE ZENTRIFUGALPUMPE SOWIE ZENTRIFUGALPUMPE**

(30) Priorität: 30.04.2024 EP 24173505
(71) Anmelder: Levitronix GmbH, 8048 Zürich (CH)
(72) Erfinder: Steinert, Daniel, 8180 Bülach (CH); Hu, Rennan, 8057 Zürich (CH); Schmid, Alexander, 8915 Hausen am Albis (CH); Barletta, Natale, 8048 Zürich (CH)
(74) Vertreter: IPS Irsch AG

(57) **Zusammenfassung**

Es wird eine Pumpeneinheit für eine Zentrifugalpumpe vorgeschlagen, welche die Pumpeneinheit und einen Stator (100) umfasst, der sich in einer axialen Richtung (A) von einem ersten axialen Ende (110) bis zu einem zweiten axialen Ende (120) erstreckt, wobei an dem ersten axialen Ende (110) eine becherförmige Ausnehmung vorgesehen ist, in welche die Pumpeneinheit (1) einsetzbar ist, wobei die Pumpeneinheit (1) ein Pumpengehäuse (2) mit einem Einlass (21) und mit einem Auslass (22) für ein zu förderndes Fluid aufweist, sowie einen in dem Pumpengehäuse (2) angeordneten Rotor (10) mit einer Mehrzahl von Flügeln (103) zum Fördern des Fluids, wobei der Rotor (10) um die axiale Richtung (A) rotierbar ist, wobei die Pumpeneinheit (1) für eine berührungslos magnetische Lagerung des Rotors (10) und für einen berührungslos magnetischen Antrieb des Rotors (10) durch den Stator (100) ausgestaltet ist, wobei die Flügel (103) des Rotors (10) um einen zentralen Einlassbereich (25) des Rotors (10) herum angeordnet sind, wobei der Rotor (10) mindestens eine Entlastungsöffnung (104, 104a) zur Erzeugung eines Rezirkulationsflusses (RF) umfasst, der von einer dem Einlass (21) abgewandten Rückseite des Rotors (10) in Richtung des zentralen Einlassbereichs (25) des Rotors (10) gerichtet ist, und wobei der Rotor (10) ferner ein im zentralen Einlassbereich (25) angeordnetes Trennelement (7) aufweist, welches den Rezirkulationsfluss (RF) in eine zur axialen Richtung (A) senkrechte radiale Richtung umlenkt. Die Mehrzahl von Flügeln (103), das Trennelement (7) und die mindestens eine Entlastungsöffnung (104, 104a) des Rotors (10) sind als einstückige Einheit ausgestaltet. Ferner wird eine Zentrifugalpumpe zum Fördern eines Fluids vorgeschlagen, die eine solche Pumpeneinheit (1) umfasst.

## Beschreibung

Die Erfindung betrifft eine Pumpeneinheit für eine Zentrifugalpumpe gemäss dem Oberbegriff des unabhängigen Patentanspruchs. Die Erfindung betrifft ferner eine Zentrifugalpumpe mit einer solchen Pumpeneinheit.

Es sind Zentrifugalpumpen bekannt, welche eine Pumpeneinheit und einen Stator umfassen, der als Antriebseinheit für den Rotor der Pumpeneinheit ausgestaltet ist, wobei der Rotor der Pumpeneinheit das Flügelrad der Zentrifugalpumpe bildet. Dabei ist der Rotor in der Pumpeneinheit mittels des Stators berührungslos magnetisch lagerbar und berührungslos zur Rotation um eine axiale Richtung antreibbar. Solche Zentrifugalpumpen werden beispielsweise von der Anmelderin unter der Produktbezeichnung Levitronix^{®} BPS Pumpen vertrieben.

Der Stator und der Rotor bilden einen elektromagnetischen Drehantrieb. Bei den Levitronix^{®} BPS Pumpen, beispielsweise, ist der elektromagnetische Drehantrieb nach dem Prinzip des lagerlosen Motors ausgestaltet. Mit dem Begriff lagerloser Motor ist dabei ein elektromagnetischer Drehantrieb gemeint, bei welchem der Rotor vollkommen magnetisch bezüglich des Stators lagerbar ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation um eine durch die axiale Richtung definierte Solldrehachse bewirkt, und welches zum anderen eine beliebig einstellbare Querkraft auf den Rotor ausübt, sodass dessen radiale Position aktiv steuerbar bzw. regelbar ist. Somit sind drei Freiheitsgrade des Rotors aktiv regelbar, nämlich seine Rotation sowie seine radiale Position (zwei Freiheitsgrade). Bezüglich dreier weiterer Freiheitsgrade, nämlich seiner Position in axialer Richtung und Verkippungen bezüglich der zur Solldrehachse senkrechten radialen Ebene (zwei Freiheitsgrade), ist der Rotor passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des Rotors ist die Eigenschaft, welcher der lagerlose Motor seinen Namen verdankt. In dem Lager- und Antriebsstator lässt sich die Lagerfunktion nicht von der Antriebsfunktion separieren.

Es sind natürlich auch andere Ausgestaltungen von Zentrifugalpumpen bekannt, bei welchen der Rotor berührungslos magnetisch gelagert ist, beispielsweise solche bei denen separate Magnetlager für den Rotor vorgesehen sind, sodass die magnetische Lagerfunktion von der Antriebsfunktion getrennt ist. Beispielsweise sind dazu separate Spulen vorgesehen, mit denen nur die Lagerkräfte für den Rotor realisiert werden, die aber keinen Beitrag zum Antrieb des Rotors leisten. Eine solche Zentrifugalpumpe ist beispielsweise in der WO 2022/004144 offenbart.

Zentrifugalpumpen mit berührungslos magnetisch gelagerten und angetriebenen Rotoren, beispielsweise solche, die nach dem Prinzip des lagerlosen Motors ausgestaltet und betrieben werden, haben sich in einer Vielzahl von Anwendungen bewährt. Aufgrund der Abwesenheit von mechanischen Lagern eignen sich solche Zentrifugalpumpen für Anwendungen, bei denen sehr empfindliche Substanzen gefördert werden, beispielsweise Blutpumpen, oder bei denen sehr hohe Anforderungen an die Reinheit gestellt werden, beispielsweise in der Halbleiterindustrie, der pharmazeutischen Industrie, der biotechnologischen Industrie, oder bei denen abrasive oder aggressive Substanzen gefördert werden, welche mechanische Lager sehr schnell zerstören würden, beispielsweise Pumpen für Slurry, Schwefel-, Phosphorsäure oder andere Chemikalien in der Halbleiterindustrie.

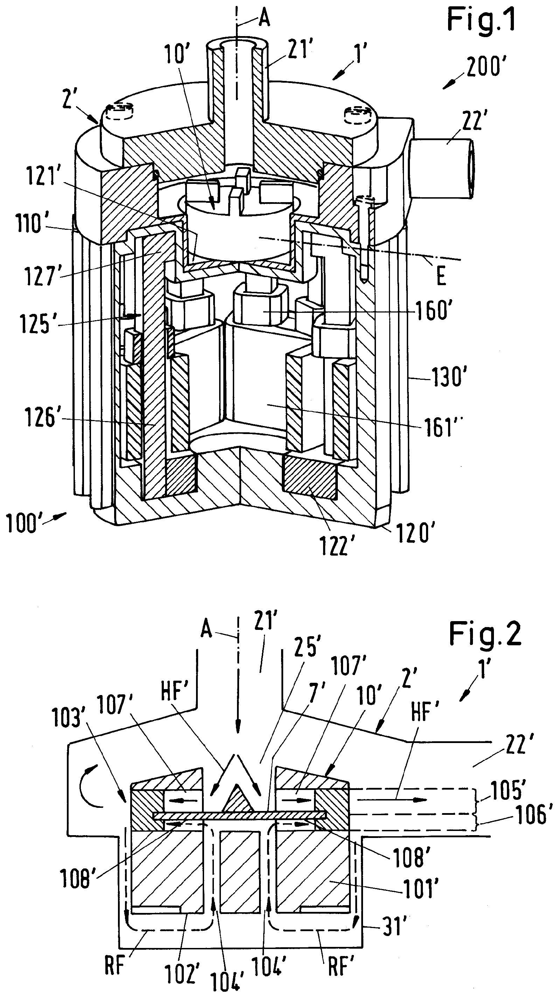

Fig. 1 zeigt eine Darstellung einer aus dem Stand der Technik bekannten Zentrifugalpumpe 200', die nach dem Prinzip des lagerlosen Motors ausgestaltet ist. Es handelt sich beispielsweise um eine Levitronix^{®} BPS Pumpe. Zum besseren Verständnis ist in Fig. 1 ein Segment herausgeschnitten, damit das Innere der Zentrifugalpumpe 200' sichtbar ist. Die Zentrifugalpumpe 200' umfasst einen Stator 100' und eine Pumpeneinheit 1'.

Eine Pumpeneinheit 1', die für diesen Typ von Zentrifugalpumpen 200' geeignet ist, wird beispielsweise in der EP 2 273 124 A1 offenbart. Fig. 2 zeigt eine solche Pumpeneinheit 1' in einer Schnittdarstellung, wobei der Schnitt in axialer Richtung A erfolgt.

Um anzuzeigen, dass es sich bei der Darstellung in Fig. 1 und Fig. 2 um Vorrichtungen aus dem Stand der Technik handelt, sind hier die Bezugszeichen jeweils mit einem Hochkomma bzw. mit einem Strich versehen. Die Zentrifugalpumpe ist gesamthaft mit dem Bezugszeichen 200' bezeichnet.

In der Pumpeneinheit 1' ist ein Rotor 10' angeordnet, welcher das Flügelrad bzw. das Laufrad bildet, mit dem das Fluid gefördert wird. Der Stator 100' hat ein Statorgehäuse 130' und erstreckt sich in einer axialen Richtung A von einem ersten axialen Ende 110' bis zu einem zweiten axialen Ende 120', wobei an dem ersten axialen Ende 110' eine becherförmige Ausnehmung 121' vorgesehen ist, in welche die Pumpeneinheit 1' einsetzbar ist. Der Stator 100' bildet mit dem Rotor 10' einen elektromagnetischen Drehantrieb zum Rotieren des Rotors 10' um die axiale Richtung A. Der Stator 100' ist zur berührungslosen magnetischen Lagerung des Rotors 10' nach dem Prinzip des lagerlosen Motors ausgestaltet. Hierzu ist der Stator 100' als Lager- und Antriebsstator ausgestaltet, mit welchem der Rotor 10' berührungslos magnetisch zur Rotation um die axiale Richtung A antreibbar und berührungslos magnetisch bezüglich des Stators 100' lagerbar ist, wobei der Rotor 10' bezüglich der axialen Richtung A passiv magnetisch stabilisiert ist, und in einer zur axialen Richtung A senkrechten radialen Ebene, die in Fig. 1 durch die Linie E angedeutet ist, aktiv magnetisch gelagert ist.

Der elektromagnetische Drehantrieb mit dem Stator 100' und dem Rotor 10' ist als sogenannter Tempelmotor ausgestaltet. Der Stator 100' umfasst eine Mehrzahl von Spulenkernen 125', hier acht Spulenkerne 125', von denen jeder einen Längsschenkel 126' umfasst, welcher sich von einem ersten Ende, in Fig. 1 das darstellungsgemäss untere Ende, in axialer Richtung A bis zu einem zweiten Ende erstreckt, sowie einen Querschenkel 127', welcher an dem zweiten Ende des Längsschenkels 126' und in der radialen Ebene E angeordnet ist. Jeder Querschenkel 127' erstreckt sich von dem zugehörigen Längsschenkel 126' in radialer Richtung auf den Rotor 10' zu und wird durch eine radial innenliegende Stirnfläche begrenzt. Die Spulenkerne 126' sind bezüglich der Umfangsrichtung um die becherförmige Ausnehmung 121' herum angeordnet und damit um den Rotor 10' herum, sodass der Rotor 10' zwischen den radial innenliegenden Stirnflächen der Querschenkel 127' der Spulenkerne 126' angeordnet ist.

Alle ersten Enden der Längsschenkel 126' sind durch einen Rückschluss 122' zur Führung des magnetischen Flusses miteinander verbunden. An jedem Längsschenkel 126' ist mindestens eine konzentrierte Wicklung 160', 161' vorgesehen, welche den jeweiligen Längsschenkel 126' umgibt. Bezüglich Anzahl und Anordnung der konzentrierten Wicklungen 160', 161' sind zahlreiche Varianten bekannt, die hier nicht näher erläutert werden. Es gibt beispielsweise solche Wicklungen 160', die um genau einen Längsschenkel 126' herum gewickelt sind, und solche Wicklungen 161', die um genau zwei Längsschenkel 126' herum angeordnet sind.

Die Mehrzahl der Längsschenkel 126', die sich in axialer Richtung A erstrecken und an die Säulen eines Tempels erinnern, hat dem Tempelmotor seinen Namen gegeben.

Die Pumpeneinheit 1', die aus der EP 2 273 124 A1 bekannt ist (Fig. 2), umfasst ein Pumpengehäuse 2' mit einem Einlass 21' und mit einem Auslass 22' für das zu fördernde Fluid, sowie den in dem Pumpengehäuse 2' angeordneten Rotor 10' zum Fördern des Fluids, der um die axiale Richtung A rotierbar ist. Der Rotor 10' umfasst einen magnetisch wirksamen Kern 101', welcher für die Drehmomentbildung sowie für die Erzeugung der magnetischen Lagerkräfte magnetisch mit dem Stator 100' zusammenwirkt. Der magnetisch wirksame Kern 101' ist beispielsweise ein permanentmagnetischer Ring oder eine permanentmagnetische Scheibe.

Es sind auch solche Ausgestaltungen möglich, bei denen der magnetisch wirksame Kern 101' permanentmagnetfrei, also ohne Permanentmagnete ausgestaltet ist. Der Rotor 10' ist dann z.B. als Reluktanzläufer ausgestaltet. Der magnetisch wirksame Kern 101' des Rotors 10' besteht dann beispielsweise aus einem weichmagnetischen Material. Geeignete weichmagnetische Materialien für den magnetisch wirksamen Kern 101' sind beispielsweise ferromagnetische oder ferrimagnetische Materialien, also insbesondere Eisen, Nickel-Eisen, Kobalt-Eisen, Silizium-Eisen, Mu-Metall.

Ferner sind Ausgestaltungen möglich, bei denen der magnetisch wirksame Kern 101' des Rotors 10' sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Typischerweise ist der magnetisch wirksame Kern 101' mit einem Kunststoff vollständig ummantelt. In anderen Ausführungsformen ist der magnetisch wirksame Kern 101' vollständig in einer Ummantelung 102' eingeschlossen, die aus einem keramischen Material oder aus einem metallischen Material, beispielsweise einem rostfreien Stahl oder Titan oder Tantal, besteht.

Der Rotor 10' umfasst ferner eine Mehrzahl von Flügeln 103' zum Fördern des Fluids vom Einlass 21' zum Auslass 22'.

Der Einlass 21' des Pumpengehäuses 2' ist derart angeordnet und ausgestaltet, dass das zu fördernde Fluid den Rotor 10' in axialer Richtung A anströmt. Der Auslass 22'erstreckt sich parallel zur radialen Ebene E, also im Wesentlichen senkrecht zum Einlass 21'.

Das Pumpengehäuses 2' umfasst einen zylindrischen Becher 31' zur Aufnahme des Rotors 10'. Der Becher 31' wird in die Ausnehmung 121' im Statorgehäuse 130' eingesetzt, sodass der Rotor 10', genauer gesagt der magnetisch wirksame Kern 101' des Rotors 10' zwischen den Querschenkeln 127' der Spulenkerne 126' angeordnet ist.

Für viele Anwendungen, beispielsweise für Anwendungen in der Halbleiterindustrie, wird die Pumpeneinheit 1' - mit Ausnahme des magnetisch wirksamen Kerns 101' - aus einem Kunststoff gefertigt, beispielsweise aus einem Perfluoralkoxy-Polymer (PFA) oder aus Polytetrafluorethylen (PTFE), weil dies Kunststoffe mit einer besonders hohen chemischen Resistenz sind. Diese Kunststoffe sind praktisch inerte Stoffe, die auch von chemisch sehr aggressiven Substanzen, wie sie häufig in der Halbleiterindustrie zum Einsatz kommen, nicht angegriffen werden können. Zudem sind PFA und PTFE sehr reine Kunststoffe, weil sie üblicherweise keine Zusatzstoffe aufweisen und ihre Molekülkomplexe zumindest näherungsweise inert sind. PFA ist häufig bevorzugt, weil es in Spritzgiessverfahren verarbeitet werden kann.

In Zentrifugalpumpen 200', bei denen das zu fördernde Fluid aus der axialen Richtung A in eine radiale Richtung umgelenkt wird, erfährt der Rotor 10'starke Belastungen in der axialen Richtung A. Der axiale Schub, der auf den Rotor wirkt, wird vor allem durch die Druckdifferenz am Rotor 10' verursacht. Während auf der dem Einlass 21' zugewandten Seite des Rotors 10' im Wesentlichen der Saugdruck herrscht, liegt auf der Rückseite des Rotors 10' ein höherer Druck an, denn die Rotorrückseite steht mit dem Auslass 22' in Verbindung, wo im Wesentlichen der Förderdruck herrscht. Der hieraus resultierende Axialschub stellt insbesondere in Zentrifugalpumpen 200' mit berührungslos magnetisch gelagertem Rotor 10' eine Herausforderung dar. Damit der Axialschub nicht vollständig von der axialen magnetischen Lagerung bzw. Stabilisierung des Rotors 10' aufgenommen werden muss, sind verschiedene Massnahmen bekannt, beispielsweise Entlastungsöffnungen 104', die sich in axialer Richtung A durch den gesamten Rotor 10' hindurch erstrecken, und so eine Strömungsverbindung zwischen der dem Einlass 21' zugewandten Vorderseite des Rotors 10' und seiner Rückseite bilden, was zu einer Druckentlastung des Rotors 10' bezüglich der axialen Richtung A führt.

In der EP 2 273 124 wird beispielsweise vorgeschlagen, die Flügel 103' des Rotors 10' durch ein senkrecht zur axialen Richtung A ausgerichtetes Trennelement 7' in zwei Flügelräder aufzuteilen, nämlich in ein erstes Flügelrad 105' zur Erzeugung eines Hauptflusses HF' vom Einlass 21' zum Auslass 22', und ein zweites Flügelrad 106' zur Erzeugung eines Rezirkulationsflusses RF', der von der Rückseite des Rotors 10' durch die Entlastungsöffnungen 104' gerichtet ist. Der Hauptfluss HF' ist in Fig. 2 mit durchgezogenen Pfeilen HF' angedeutet, während der Rezirkulationsfluss RF' mit gestrichelten Pfeilen RF' angedeutet ist. Durch das Trennelement 7' wird jeder Flügel 103' in einen ersten Flügel 107' und einen zweiten Flügel 108' aufgeteilt. Die Gesamtheit der ersten Flügel 107' bilden das erste Flügelrad 105', und die Gesamtheit der zweiten Flügel 108' bilden das zweite Flügelrad 108'. Die ersten Flügel 107' sind so angeordnet, dass ein zentraler Einlassbereich 25' des Rotors 10' frei von Flügeln 103' ist. Die Flügel 103' sind um diesen zentralen Einlassbereich 25' herum angeordnet.

Das Trennelement 7', welches die beiden Flügelräder 105' und 106' voneinander trennt, lenkt den Rezirkulationsfluss RF' aus der axialen Richtung A in die radiale Richtung um, und trennt zumindest teilweise den Rezirkulationsfluss RF' vom Hauptfluss HF', sodass diese sich nicht unmittelbar am Ausgang der Entlastungsöffnungen 104' miteinander vermischen können. Dabei erstreckt sich das Trennelement 7' bezüglich der radialen Richtung bis in die Flügel 103' hinein, das heisst in radialer Richtung überlappt das Trennelement 7' mit den Flügeln 103'

Auch wenn sich diese Ausgestaltung mit dem Trennelement 7' in der Praxis bewährt hat, so ist die Herstellung eines solchen Rotors10' sehr komplex und aufwändig. So ist es beispielsweise notwendig, den Rotor 10' aus mehreren Einzelteilen zusammenzusetzten. Für das Trennelement 7' müssen in den Flügeln 103' Ausnehmungen vorgesehen werden, sodass das Trennelement 7' zwischen den Flügeln 103' eingesetzt werden kann.

Wenn die Pumpeneinheit 1' beispielsweise aus einem Kunststoff hergestellt wird, so müssen die einzelnen Komponenten zuverlässig und stabil miteinander verbunden werden. Dies erfolgt beispielsweise durch Schweissprozesse. Neben dem Zeit- und dem Kostenaufwand bringt jeder Schweissprozess das Risiko mit sich, dass an der Schweissverbindung Leckagen auftreten, wodurch die Betriebssicherheit der gesamten Zentrifugalpumpe 200' gefährdet wird. Auch besteht die Gefahr, dass an den Schweissverbindungen Risse oder kleine Spalte entstehen können. In diesen können sich Verschmutzungen ablagern, welche sich dann im Betriebszustand ablösen und das zu fördernde Fluid verunreinigen. In vielen Anwendungen, beispielsweise in der Halbleiterindustrie, können aber bereits kleinste Verunreinigungen drastische Folgen haben, beispielsweise das Endprodukt unbrauchbar machen.

Damit die fluidberührenden Komponenten des Rotors 10', insbesondere die axialen Entlastungsöffnungen 104' und auch die innen liegenden Kanten unterhalb des Trennelements 7', ihre beabsichtigte Funktion erfüllen können, ist eine extrem hohe Präzision und Masshaltigkeit der Komponenten von Nöten. In den meisten Fällen werden die Komponenten mit Herstellungsverfahren (z.B. Spritzgiessverfahren) hergestellt, bei welchen Abweichungen von einer vorgegebenen Soll-Geometrie unvermeidbar sind. Dies führt dazu, dass überstehende Ausbuchtungen oder andere Abweichungen von der Soll-Geometrie die Fluidströmung stören und somit die Funktion deutlich verschlechtern. Das heisst, es ist notwendig, dass die Komponenten vor dem Zusammensetzen, also in den Einzelteilen, nachbearbeitet werden müssen. Wenn die Komponenten zum Rotor 10' bereits zusammengesetzt sind, ist eine Nachbearbeitung nicht mehr möglich, da die Bereiche des Rotors 10', an denen eine Nachbearbeitung notwendig ist, für die entsprechenden Nachbearbeitungswerkzeuge nicht mehr ausreichend zugänglich sind. Das heisst, einerseits, dass die aus dem Stand der Technik bekannten Rotoren 10' aus mehreren Einzelteilen zusammengesetzt sein müssen und andererseits, dass abgesehen vom Zusammensetzen des Rotors 10'zusätzliche Arbeitsschritte notwendig sind, damit der Rotor 10' seine vorgesehenen Funktionen erfüllen kann.

Ausgehend von diesem Stand der Technik ist es daher eine Aufgabe der Erfindung, eine Pumpeneinheit mit einem berührungslos magnetisch lagerbaren Rotor für eine Zentrifugalpumpe vorzuschlagen, die besonders einfach hinsichtlich der Herstellung ist, und die sich durch eine hohe Betriebssicherheit auszeichnet. Zudem ist es eine Aufgabe der Erfindung, eine Zentrifugalpumpe mit einer solchen Pumpeneinheit vorzuschlagen.

Der diese Aufgabe lösende Gegenstand der Erfindung ist durch die Merkmale des unabhängigen Patentanspruchs gekennzeichnet.

Erfindungsgemäss wird also eine Pumpeneinheit für eine Zentrifugalpumpe vorgeschlagen, welche die Pumpeneinheit und einen Stator umfasst, der sich in einer axialen Richtung von einem ersten axialen Ende bis zu einem zweiten axialen Ende erstreckt, wobei an dem ersten axialen Ende eine becherförmige Ausnehmung vorgesehen ist, in welche die Pumpeneinheit einsetzbar ist, wobei die Pumpeneinheit ein Pumpengehäuse mit einem Einlass und mit einem Auslass für ein zu förderndes Fluid aufweist, sowie einen in dem Pumpengehäuse angeordneten Rotor mit einer Mehrzahl von Flügeln zum Fördern des Fluids, wobei der Rotor um die axiale Richtung rotierbar ist, wobei die Pumpeneinheit für eine berührungslos magnetische Lagerung des Rotors und für einen berührungslos magnetischen Antrieb des Rotors durch den Stator ausgestaltet ist, wobei die Flügel des Rotors um einen zentralen Einlassbereich des Rotors herum angeordnet sind, wobei der Rotor mindestens eine Entlastungsöffnung zur Erzeugung eines Rezirkulationsflusses umfasst, der von einer dem Einlass abgewandten Rückseite des Rotors in Richtung des zentralen Einlassbereichs des Rotors gerichtet ist, und wobei der Rotor ferner ein im zentralen Einlassbereich angeordnetes Trennelement aufweist, welches den Rezirkulationsfluss in eine zur axialen Richtung senkrechte radiale Richtung umlenkt. Die Mehrzahl von Flügeln, das Trennelement und die mindestens eine Entlastungsöffnung des Rotors sind als einstückige Einheit ausgestaltet.

Durch die einstückige Ausgestaltung der Einheit aus den Flügeln, dem Trennelement und der mindestens einen Entlastungsöffnung des Rotors muss der Rotor nicht mehr aus mehreren Komponenten zusammengebaut werden, sondern kann als monolithische Vorrichtung in sehr einfacher Weise hergestellt werden. Zudem bedarf es keiner Verbindungen einzelner Komponenten durch beispielsweise Kleben, Verschrauben oder Schweissen, wodurch sich einerseits der konstruktive Aufwand reduziert, und sich andererseits die Betriebssicherheit erhöht, weil beispielsweise keine Schweissverbindungen mehr notwendig sind, welche im Betriebszustand zu Leckagen führen könnten.

Beispielsweise ist es möglich, die einstückige Einheit, welche die Flügel, das Trennelement und die mindestens eine Entlastungsöffnung umfasst, als einstückiges Spritzgussteil auszugestalten. Die Herstellung in einem Spritzgiessverfahren ermöglicht eine besonders kostengünstige und wirtschaftliche Herstellung des Rotors. Zudem ist der Rotor dann notwendigerweise so ausgestaltet, dass er entformbar ist, also nach dem Spritzgiessprozess aus dem Werkzeug entnommen werden kann.

Gemäss einer bevorzugten Ausgestaltung ist genau eine Entlastungsöffnung vorgesehen, welche den zentralen Einlassbereich des Rotors mit der Rückseite des Rotors verbindet. Diese Entlastungsöffnung ist dann zentral im Rotor angeordnet.

In anderen Ausgestaltungen der erfindungsgemässen Pumpeneinheit sind mehrere Entlastungsöffnungen vorgesehen, welche um eine Mittelachse des Rotors herum angeordnet sind, wobei jede Entlastungsöffnung den zentralen Einlassbereich des Rotors mit der Rückseite des Rotors verbindet. Beispielsweise sind die Entlastungsöffnungen auf einer Kreislinie angeordnet, deren Mittelpunkt auf der Mittelachse des Rotors liegt. Bei diesen Ausgestaltungen kann auch eine Entlastungsöffnung im Zentrum des Rotors vorgesehen sein, welche die Mittelachse umschliesst. Die anderen Entlastungsöffnungen sind dann um die Entlastungsöffnung im Zentrum herum angeordnet.

Vorzugsweise umfasst der Rotor einen ringförmigen oder scheibenförmigen magnetisch wirksamen Kern, sowie eine Ummantelung, welche den magnetisch wirksamen Kern vollständig umschliesst, wobei die Ummantelung ein Bestandteil der einstückigen Einheit ist, welche die Flügel und das Trennelement umfasst. Bei dieser Ausgestaltung sind die Ummantelung, die Flügel, das Trennelement und alle Entlastungsöffnungen als monolithische Komponente ausgestaltet.

In einer bevorzugten Ausführungsform ist das Trennelement derart ausgestaltet und angeordnet, dass die mindestens eine Entlastungsöffnung vom Einlass aus teilweise sichtbar ist. Das bedeutet, dass das Trennelement die Entlastungsöffnung oder die Entlastungsöffnungen nicht vollständig überdeckt. Dies hat den Vorteil, dass die Entlastungsöffnung(en) vom Pumpeneinlass aus zugänglich ist (sind), wodurch beispielsweise eine Bearbeitung der Entlastungsöffnung (en), beispielsweise eine spanabhebende Nachbearbeitung ermöglicht wird.

Gemäss einer bevorzugten Ausgestaltung umfasst das Trennelement eine Trennplatte und Befestigungsstege, wobei die Trennplatte einen maximalen Aussendurchmesser in radialer Richtung aufweist, der höchstens so gross ist wie der Durchmesser des zentralen Einlassbereichs des Rotors, und wobei die Befestigungsstege zum Fixieren der Trennplatte ausgestaltet sind. Durch die Ausgestaltung mit den Befestigungsstegen ist es nicht mehr notwendig, aber dennoch möglich, dass das Trennelement an den Flügeln befestigt wird, wodurch sich der konstruktive Aufwand reduziert.

Vorzugsweise ist die Trennplatte so ausgestaltet, dass der maximale Aussendurchmesser der Trennplatte kleiner ist als der Durchmesser des zentralen Einlassbereichs des Rotors. Die Trennplatte ist dann also bezüglich der radialen Richtung so bemessen, dass sie zwischen den Flügeln angeordnet werden kann, ohne die Flügel zu berühren.

Eine weitere bevorzugte Ausgestaltung besteht darin, dass sich jeder Befestigungssteg von der Trennplatte bis zu der Ummantelung erstreckt, wobei zwischen benachbarten Befestigungsstegen jeweils eine radiale Öffnung für den Rezirkulationsfluss vorgesehen ist. Hierdurch ist die Trennplatte an der Ummantelung fixiert, wobei der Rezirkulationsfluss zwischen den Befestigungsstegen in radialer Richtung ausströmen kann. Dabei ist es bevorzugt, dass die radialen Öffnungen zwischen den Befestigungsstegen so angeordnet sind, dass sie in radialer Richtung gesehen mit den Zwischenräumen zwischen zwei benachbarten Flügeln fluchten, sodass der Rezirkulationsfluss ungehindert zwischen zwei benachbarten Flügeln einströmen kann.

Eine bevorzugte Ausführungsform besteht darin, dass sich jeder Befestigungssteg von einer Unterseite der Trennplatte in axialer Richtung bis zu der Ummantelung erstreckt. Bei dieser Ausführungsform werden die Befestigungsstege vorzugsweise vollständig von der Trennplatte überdeckt, sodass die Trennstege vom Einlass aus nicht sichtbar sind.

Gemäss einer anderen bevorzugten Ausführungsform ist jeder Befestigungssteg am Aussenrand der Trennplatte angeordnet, und erstreckt sich vom Aussenrand in radialer Richtung. Bei dieser Ausgestaltung der Befestigungsstege als radiale Verstrebungen sind die Befestigungsstege vom Einlass aus sichtbar. Vom Einlass des Pumpengehäuses betrachtet sieht das Trennelement dann sternförmig aus.

Bei den Ausgestaltungen, bei denen die Befestigungsstege am Aussenrand der Trennplatte angeordnet sind, ist es bevorzugt, dass die Befestigungsstege äquidistant am Aussenrand der Trennplatte angeordnet sind.

Eine bevorzugte Variante dieser Ausgestaltung besteht darin, dass sich jeder Befestigungssteg in radialer Richtung jeweils bis an einen der Flügel erstreckt. Jeder der Befestigungsstege steht dann in direktem körperlichem Kontakt mit einem der Flügel. Diese Ausgestaltung hat auch den Vorteil, dass die radialen Öffnungen für den Rezirkulationsfluss, die zwischen den Befestigungsstegen angeordnet sind, in die radialen Öffnungen zwischen benachbarten Flügel übergehen, wodurch insbesondere Verwirbelung vermieden oder zumindest drastisch reduziert werden können.

Ferner ist es insbesondere bei diesen Ausgestaltungen bevorzugt, dass die Anzahl der Befestigungsstege gleich der Anzahl der Flügel ist. Hierdurch werden durchgängige Kanäle für den Rezirkulationsfluss gebildet.

Durch die Erfindung wird ferner eine Zentrifugalpumpe zum Fördern eines Fluids vorgeschlagen mit einer Pumpeneinheit, die nach einem der vorangehenden Ansprüche ausgestaltet ist, und die einen zylindrischen Becher zur Aufnahme des Rotors aufweist, sowie mit einem Stator, der sich in einer axialen Richtung von einem ersten axialen Ende bis zu einem zweiten axialen Ende erstreckt, wobei an dem ersten axialen Ende eine becherförmige Ausnehmung vorgesehen ist, in welche der zylindrischen Becher der Pumpeneinheit einsetzbar ist, wobei der Stator mit dem Rotor einen elektromagnetischen Drehantrieb zum Rotieren des Rotors um die axiale Richtung bildet, wobei der Stator als Lager- und Antriebsstator ausgestaltet ist, mit welchem der Rotor berührungslos magnetisch antreibbar und berührungslos magnetisch bezüglich des Stators lagerbar ist, wobei der Rotor bezüglich der axialen Richtung passiv magnetisch stabilisiert ist, und in einer zur axialen Richtung senkrechten radialen Ebene aktiv magnetisch gelagert ist.

Besonders bevorzugt ist der elektromagnetische Drehantrieb als Tempelmotor ausgestaltet ist, wobei der Stator eine Mehrzahl von Spulenkernen aufweist, von denen jeder einen Längsschenkel umfasst, welcher sich von einem ersten Ende in axialer Richtung bis zu einem zweiten Ende erstreckt, sowie einen Querschenkel, welcher an dem zweiten Ende des Längsschenkels und in der radialen Ebene angeordnet ist, und welcher sich von dem Längsschenkel in radialer Richtung erstreckt, wobei die Spulenkerne bezüglich der Umfangsrichtung um den Rotor herum angeordnet sind, sodass der Rotor zwischen den Querschenkeln der Spulenkerne angeordnet ist, und wobei an jedem Längsschenkel mindestens eine konzentrierte Wicklung vorgesehen ist, welche den jeweiligen Längsschenkel umgibt.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der schematischen Zeichnung zeigen (teilweise im Schnitt):
- Fig. 1:: eine perspektivische Darstellung einer Zentrifugalpumpe gemäss Stand der Technik, teilweise im Schnitt,
- Fig. 2:: eine Pumpeneinheit gemäss Stand der Technik in einer Schnittdarstellung,
- Fig. 3: ein Ausführungsbeispiel einer erfindungsgemässen Pumpeneinheit in einer Schnittdarstellung,
- Fig. 4:: eine perspektivische Darstellung des Rotors der Pumpeneinheit aus Fig. 3, teilweise im Schnitt
- Fig. 5:: eine Schnittdarstellung des Rotors in einem Schnitt entlang der Schnittlinie V-V in Fig. 4,
- Fig. 6:: eine erste Variante des Rotors in einer Schnittdarstellung,
- Fig. 7:: eine perspektivische Darstellung der ersten Variante des Rotors aus Fig. 6, teilweise im Schnitt,
- Fig. 8:: eine Schnittdarstellung der ersten Variante des Rotors in einem Schnitt entlang der Schnittlinie VIII-VIII in Fig. 7,
- Fig. 9:: eine zweite Variante des Rotors in einer Schnittdarstellung,
- Fig. 9A:: die zweite Variante des Rotors in einer Aufsicht vom Einlass des Pumpengehäuses,
- Fig. 10:: eine perspektivische Darstellung der zweiten Variante des Rotors aus Fig. 9, teilweise im Schnitt,
- Fig. 11:: eine Schnittdarstellung der zweiten Variante des Rotors in einem Schnitt entlang der Schnittlinie XI-XI in Fig. 10,
- Fig. 12:: eine dritte Variante des Rotors in einer Aufsicht vom Einlass des Pumpengehäuses,
- Fig. 13:: eine perspektivische Darstellung der dritten Variante des Rotors aus Fig. 12, teilweise im Schnitt,
- Fig. 14:: eine Schnittdarstellung der dritten Variante des Rotors in einem Schnitt entlang der Schnittlinie XIV-XIV in Fig. 13, und
- Fig. 15:: eine schematische Schnittdarstellung eines Ausführungsbeispiels einer erfindungsgemässen Zentrifugalpumpe.

Wie bereits vorangehend erläutert, zeigt Fig. 1 eine Zentrifugalpumpe 200' mit einem berührungslos magnetisch gelagerten und berührungslos magnetisch angetriebenen Rotor 10', die aus dem Stand der Technik bekannt ist. Fig. 2 zeigt in einer Schnittdarstellung eine Pumpeneinheit 1', die aus dem Stand der Technik bekannt ist, und die beispielsweise für die Zentrifugalpumpe 200' aus Fig. 1 geeignet ist.

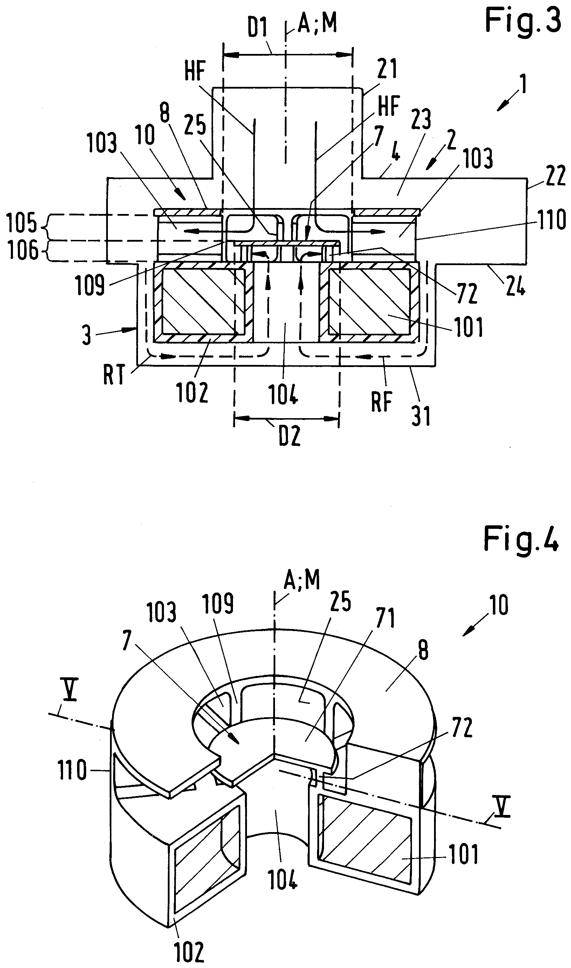

Fig. 3 zeigt in einer der Fig. 2 entsprechenden Schnittdarstellung ein Ausführungsbeispiel einer erfindungsgemässen Pumpeneinheit, die gesamthaft mit dem Bezugszeichen 1 bezeichnet ist.

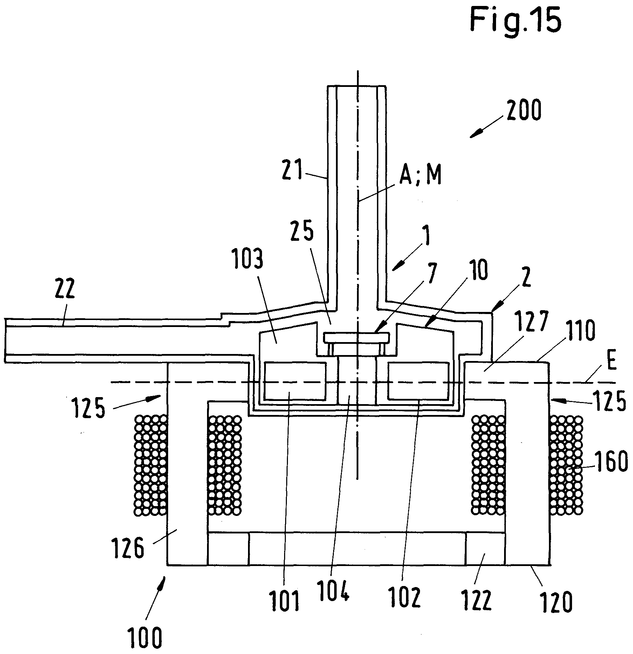

Die Pumpeneinheit 1 ist für eine Zentrifugalpumpe 200 (siehe Fig. 15) zum Fördern eines Fluids ausgestaltet und umfasst ein Pumpengehäuse 2 mit einem Einlass 21 und mit einem Auslass 22 für das Fluid. In dem Pumpengehäuse 2 ist ein Rotor 10 zum Fördern des Fluids angeordnet, welcher das Flügelrad bzw. das Laufrad der Pumpeneinheit 1 und damit der Zentrifugalpumpe 200 bildet. Der Rotor 10 ist um eine Solldrehachse rotierbar, welche eine axiale Richtung A definiert. Diese Solldrehachse ist die Mittelachse M des Rotors 10.

Bezüglich der axialen Richtung A erstreckt sich der Rotor 10 von einer dem Einlass zugewandten Vorderseite bis zu einer dem Einlass 21 abgewandten Rückseite.

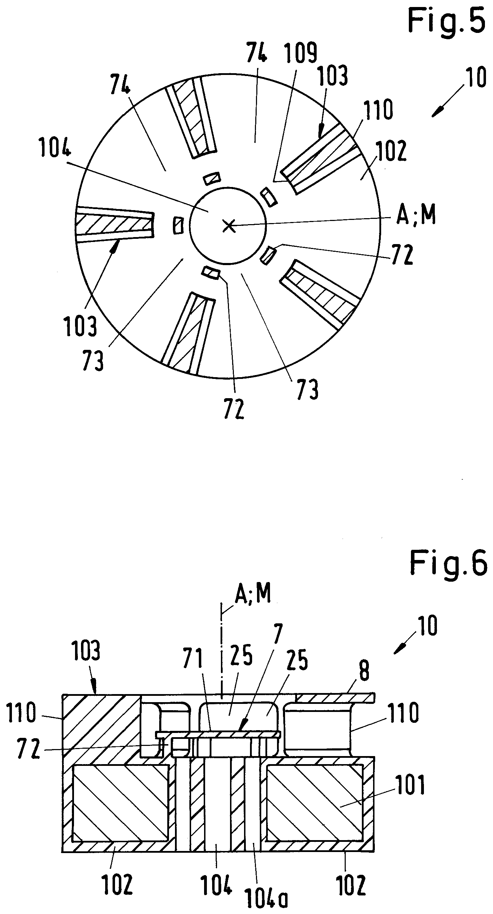

Zum besseren Verständnis zeigt Fig. 4 den Rotor 10 der Pumpeneinheit 1 in einer perspektivischen Darstellung, wobei ein Sektor aus dem Rotor 10 herausgeschnitten ist. Ferner zeigt Fig. 5 den Rotor 10 in einer Schnittdarstellung. Der Schnitt erfolgt entlang der Schnittlinie V-V in Fig. 4.

Eine zur axialen Richtung A senkrechte Richtung wird als radiale Richtung bezeichnet. Im Folgenden wird der Ausdruck "axial" mit der allgemein üblichen Bedeutung "in axialer Richtung" oder "bezüglich der axialen Richtung" verwendet. Der Begriff "radial" wird mit der allgemein üblichen Bedeutung "in radialer Richtung" oder "bezüglich der radialen Richtung" verwendet.

Die Pumpeneinheit 1 ist für eine berührungslos magnetische Lagerung des Rotors 10 und für einen berührungslos magnetischen Antrieb des Rotors 10 ausgestaltet. Dies kann insbesondere in sinngemäss gleicher Weise realisiert sein, wie dies anhand der Fig. 1 und der Fig. 2 erläutert ist. So kann die erfindungsgemässe Pumpeneinheit 1 bezüglich der magnetischen Lagerung und des magnetischen Antriebs in sinngemäss gleicher Weise ausgestaltet sein wie die Pumpeneinheit 1' in Fig. 1 oder in Fig. 2. Dazu umfasst der Rotor 10 der Pumpeneinheit 1 einen magnetisch wirksamen Kern 101, der beispielsweise als permanentmagnetischer Ring oder permanentmagnetische Scheibe ausgestaltet ist, und von einer Ummantelung 102 umschlossen wird. Die Ummantelung 102 ist vorzugsweise als Kunststoffummantelung ausgestaltet- Ummantelung 102 besteht beispielsweise aus PTFE oder PFA. Der magnetisch wirksame Kern 101 ist in der Ummantelung102 eingekapselt, das heisst, die Ummantelung 102 umschliesst den magnetisch wirksamen Kern 101 vollständig und vorzugsweise hermetisch. Dadurch ist der magnetische wirksame Kern 101 vor dem Fluid geschützt. Die Ummantelung 102 kann beispielsweise hergestellt werden, indem der magnetisch wirksame Kern 101 mit einem Kunststoff umspritzt wird.

Der magnetisch wirksamen Kern 101 des Rotors 10 ist diejenige Komponente des Rotors 10, welche für die Drehmomentbildung sowie für die Erzeugung der magnetischen Lagerkräfte magnetisch mit dem Stator 100 zusammenwirkt.

Der Rotor 10 umfasst ferner eine Mehrzahl von Flügeln 103, um das Fluid vom Einlass 21 zum Auslass 22 zu fördern. Die Flügel 103 sind auf der Ummantelung 102 des magnetisch wirksamen Kerns 101 angeordnet. Die Flügel 103 bestehen vorzugsweise aus Kunststoff und sind vorzugsweise einstückig mit der Ummantelung 102 ausgestaltet. Natürlich ist es auch möglich, die individuellen Flügel 103 oder die Gesamtheit der Flügel 103 in einem separaten Fertigungsprozess herzustellen und sie dann mit der Ummantelung 102 des magnetisch wirksamen Kerns 101 zu verbinden, beispielsweise mittels eines Schweissprozesses.

Das von dem Rotor 10 gebildete Laufrad mit den Flügeln 103 ist vorzugsweise als radiales Laufrad ausgestaltet, welches von dem Fluid vom Einlass 21 in axialer Richtung A angeströmt wird, und das Fluid dann in eine radiale Richtung umlenkt.

Das Pumpengehäuse 2 umfasst ein Deckelteil 4 und ein Bodenteil 3, die dichtend miteinander verbunden werden, was in Fig. 3 nicht näher dargestellt ist, weil es für das Verständnis der Erfindung nicht notwendig ist. Das Bodenteil 3 umfasst einen zylindrischen Becher 31 zur Aufnahme des Rotors 10. Der Becher 31 ist vorzugsweise so ausgestaltet und angeordnet, dass er in eine becherförmige Ausnehmung eines Stators 100 einsetzbar ist wie dies in Fig. 15 schematisch dargestellt ist.

Der Stator 100 (Fig. 15) erstreckt sich in der axialen Richtung A von einem ersten axialen Ende 110 bis zu einem zweiten axialen Ende 120 und weist ein Statorgehäuse (in Fig. 15 nicht dargestellt) auf, das im Wesentlichen zylindrisch ausgestaltet ist. Die becherförmige Ausnehmung ist an dem ersten axialen Ende 110 des Stators 100 angeordnet, vorzugsweise zentral in der Stirnfläche, welche das erste axiale Ende 110 des Stators 100 bildet. Die Ausgestaltung des Statorgehäuses, mit der becherförmigen Ausnehmung kann insbesondere in analoger Weise realisiert sein, wie dies anhand der Fig. 1 für das Statorgehäuse 130' und die becherförmige Ausnehmung 121' erläutert wurde. Der Becher 31 ist dann also derart angeordnet und ausgestaltet, dass er in die Ausnehmung 121' (Fig. 1) im ersten axialen Ende 110' des Stators 100' einsetzbar ist, und der magnetisch wirksame Kern 101 zwischen den Querschenkeln 127' der Spulenkerne 125' angeordnet ist.

Wie dies in Fig. 3 zu erkennen ist, befindet sich darstellungsgemäss oberhalb des Bechers 31 ein Pumpenraum 23, welcher durch das Pumpengehäuse 2 begrenzt wird, und in welchem die Flügel 103 des Rotors 10 angeordnet sind. Der Pumpenraum 23 ist zumindest im Wesentlichen zylindrisch ausgestaltet, wobei der Durchmesser des Pumpenraums 23 grösser ist als der Innendurchmesser des Bechers 31. Hierdurch weist das Pumpengehäuse 2 einen flanschartigen Vorsprung 24 auf, welcher den Pumpenraum 23 bezüglich der axialen Richtung A darstellungsgemäss nach unten begrenzt.

Der Einlass 21 ist zentral im Deckelteil 4 des Pumpengehäuses 2 angeordnet, sodass das Fluid den Rotor 10 in axialer Richtung A anströmen kann.

Jeder Flügel 103 erstreckt sich von einer radial innenliegend angeordneten Eintrittskante 109 (leading edge) bis zu einer radial aussenliegend angeordneten Austrittskante 110 (trailing edge). Bei dem hier beschriebenen Ausführungsbeispiel sind die Flügel 103 mit beispielhaftem Charakter so ausgestaltet, dass sie sich geradlinig in radialer Richtung von der Eintrittskante 109 bis zu der Austrittskante 110 erstrecken und über ihre gesamte Erstreckung eine konstante Höhe aufweisen. Mit der Höhe ist dabei die Erstreckung der Flügel 103 in axialer Richtung A gemeint. Diese Ausgestaltung ist - wie gesagt - nur beispielhaft zu verstehen. In anderen Ausführungsbeispielen sind die Flügel 103 z. B. bezüglich der radialen Richtung gekrümmt ausgestaltet und/oder mit einer Höhe in axialer Richtung A, die sich von der Eintrittskante 109 bis zur Austrittskante 110 ändert. In Fig. 15 ist beispielsweise eine Ausgestaltung gezeigt, bei welcher die Flügel 103 an ihrer Eintrittskante 109 eine grössere Höhe aufweisen als an ihrer Austrittskante 110.

Ferner ist eine ringförmige Deckplatte 8 vorgesehen, welche auf den dem Einlass 21 zugewandten Oberkanten der Flügel 103 angeordnet ist. Die Deckplatte 8 überdeckt alle Flügel 103. Bezüglich der radialen Richtung erstreckt sich die ringförmige Deckplatte 8 von den Eintrittskanten 109 der Flügel 103 bis zu ihren Austrittskanten 110. Die Deckplatte 8 kann einstückig mit den Flügeln 103 ausgestaltet sein.

Wie dies insbesondere in den Fig. 4 und Fig. 5 gut zu erkennen ist, sind die Flügel 103 des Rotors 10 um einen zentralen Einlassbereich 25 herum angeordnet, der frei von Flügeln 103 ist. Die Eintrittskanten 109 der Flügel 103 liegen auf einer Linie, hier einer Kreislinie, die einen von null verschiedenen Abstand von der Mittelachse M des Rotors 10 hat. Der Durchmesser D1 des zentralen Einlassbereiches 25 ist durch den Abstand der Eintrittskanten 109 der Flügel 103 von der Mittelachse M des Rotors 10 festgelegt. Dieser Durchmesser D1 des zentralen Einlassbereichs 25 ist gleich dem doppelten Abstand der Eintrittskanten 109 der Flügel 103 von der Mittelachse des Rotors 10. Bei dem hier beschriebenen Ausführungsbeispiel ist der Durchmesser D1 des zentralen Einlassbereichs 25 gleich gross wie der Innendurchmesser der ringförmigen Deckplatte 8.

Falls die Eintrittskanten 109 der Flügel nicht parallel zur axialen Richtung A verlaufen, sondern beispielsweise gegenüber der axialen Richtung A geneigt sind, so ist der Durchmesser D1 des zentralen Einlassbereichs 25 durch den Abstand der Eintrittskanten 109 an den dem Einlass 21 zugewandten Oberkanten der Flügel 103 festgelegt.

Der Rotor 10 umfasst ferner mindestens eine Entlastungsöffnung 104 zur Erzeugung eines Rezirkulationsflusses RF, der von der dem Einlass 21 abgewandten Rückseite des Rotors 10 in Richtung auf den zentralen Einlassbereich 25 hin gerichtet ist. Bei dem in den Fig. 3 bis Fig. 5 dargestellten Ausführungsbeispiel ist genau eine Entlastungsöffnung 104 vorgesehen, die kreiszylinderförmig ausgestaltet ist und sich in axialer Richtung A erstreckt. Die Entlastungsöffnung 104 ist zentral im Rotor 10 angeordnet ist, sodass die Achse dieser Entlastungsöffnung 104 mit der Mittelachse M des Rotors 10 zusammenfällt. Die Entlastungsöffnung 104 erstreckt sich von dem zentralen Einlassbereich 25 in axialer Richtung A durch den Rotor 10 hindurch bis an die Rückseite des Rotors 10. Der Rotor 10 umfasst ferner ein Trennelement 7, das im zentralen Einlassbereich 25 des Rotors 10 angeordnet ist, und welches den Rezirkulationsfluss RF, der von der Rückseite des Rotors 10 durch die Entlastungsöffnung 104 strömt aus der axialen Richtung A in die radiale Richtung umlenkt.

Vorzugsweise umfasst das Trennelement 7 eine Trennplatte 71, welche senkrecht zur axialen Richtung A ausgerichtet ist, und eine Mehrzahl von Befestigungsstegen 72 zum Fixieren der Trennplatte 71. Die Trennplatte 71 ist hier kreisscheibenförmig ausgestaltet und hat einen Aussendurchmesser D2. In anderen Ausgestaltungen kann die Trennplatte 71 auch eine von einer Kreisscheibe abweichende Form aufweisen und/oder Strömungsleitelemente haben. Dann ist mit dem Aussendurchmesser D2 der maximale Aussendurchmesser D2 gemeint, also die maximale Erstreckung der Trennplatte 71 in der radialen Richtung.

Die Trennplatte 71 ist in dem zentralen Einlassbereich 25 bezüglich der radialen Richtung zentriert angeordnet, das heisst, der Mittelpunkt der Trennplatte 71 liegt auf der Mittelachse M des Rotors 10. Bezüglich der axialen Richtung A ist die Trennplatte 71 so angeordnet, dass bei jedem Flügel 103 ein oberer Teil der Eintrittskante 109 darstellungsgemäss (Fig. 3, Fig. 4) bezüglich der axialen Richtung A oberhalb der Trennplatte 71 angeordnet ist, und ein unterer Teil der Eintrittskante 109 bezüglich der axialen Richtung A unterhalb der Trennplatte 71.

Die Gesamtheit der bezüglich der axialen Richtung A oberhalb der Trennplatte 71 angeordneten Bereiche der Flügel 103 bilden ein erstes Flügelrad 105, welches primär dazu dient, den Hauptfluss HF zu generieren, welcher aus der axialen Richtung A vom Einlass 21 kommend zum Auslass 22 strömt. Der Hauptfluss HF ist mit den durchgezogen dargestellten Pfeilen HF angedeutet.

Die Gesamtheit der bezüglich der axialen Richtung A unterhalb der Trennplatte 71 angeordneten Bereiche der Flügel 103 bilden ein zweites Flügelrad 106, welches primär dazu dient, den Rezirkulationsfluss RF zu generieren, welcher von der dem Einlass 21 abgewandten Rückseite des Rotors 10 durch die Entlastungsöffnung 104 in Richtung des zentralen Einlassbereichs 25 gerichtet ist. Der Rezirkulationsfluss RF ist mit den gestrichelt dargestellten Pfeilen RF angedeutet.

Das Trennelement 7 lenkt den Rezirkulationsfluss RF aus der axialen Richtung A in die radiale Richtung um. Das Trennelement 7 verhindert ein direktes Aufeinanderprallen bzw. einen direkten Kontakt des Hauptflusses HF mit dem Rezirkulationsfluss RF im Bereich desjenigen Endes der Entlastungsöffnung 104, das dem zentralen Einlassbereich 25 zugewandt ist. Durch das Trennelement 7 wird somit vermieden, dass der Rezirkulationsfluss RF und der Hauptfluss HF frontal - das heisst als entgegengesetzt zueinander gerichtete Strömungen - aufeinandertreffen. Durch das Trennelement 7 wird somit der Rezirkulationsfluss RF im Bereich des zentralen Einlassbereichs 25 zumindest teilweise vom Hauptfluss HF getrennt. Der Rezirkulationsfluss RF wird durch das Trennelement 7 zunächst in die radiale Richtung umgelenkt. Eine wesentliche Durchmischung des Hauptflusses HF und des Rezirkulationsflusses RF findet frühestens dann statt, wenn der Rezirkulationsfluss RF den Aussenrand der Trennplatte 71 passiert. Mit dem Aussenrand der Trennplatte 71 ist dabei der radial äusseren Rand der Trennplatte 71 gemeint. Da der Rezirkulationsfluss RF dann bereits in die radiale Richtung umgelenkt wurde, kann er sich weitgehend frei von starken Verwirbelungen mit dem Hauptfluss HF vermischen.

Wie dies am besten in Fig. 3 zu erkennen ist, ist der Aussendurchmesser D2 der Trennplatte 71 kleiner als der Durchmesser D1 des zentralen Einlassbereichs 25. Bei dieser Ausgestaltung liegt die Trennplatte 71 vollständig im zentralen Einlassbereich 25 und hat keinen körperlichen Kontakt mit den Flügeln 103. Die Eintrittskanten 109 der Flügel 103 sind um die Trennplatte 71 herum angeordnet, ohne diese zu berühren. Diese Ausgestaltung hat den Vorteil, dass die Trennplatte 71 in einfacher Weise bearbeitet werden kann, beispielsweise bei der Herstellung des Rotors 10.

Der Aussendurchmesser D2 der Trennplatte 71 ist grösser als der Innendurchmesser der Entlastungsöffnung 104, sodass die Entlastungsöffnung 104 von der Trennplatte 71 vollständig überdeckt wird. Somit ist die Entlastungsöffnung 104 vom Einlass 21 aus nicht sichtbar.

Es sind aber auch solche Ausgestaltungen möglich, bei welchen die Entlastungsöffnung 104 teilweise vom Einlass 21 aus sichtbar ist, bei welchen also die Trennplatte 71 die Entlastungsöffnung 104, nicht vollständig überdeckt. Dies kann beispielsweise so realisiert werden, dass der Aussendurchmesser D2 der Trennplatte 71 kleiner ist als der Innendurchmesser der Entlastungsöffnung 104.

Zur Fixierung der Trennplatte 71 umfasst das Trennelement 7 die Befestigungsstege 72. Alle Befestigungsstege 72 sind an der Unterseite der Trennplatte 71 angeordnet. Mit der Unterseite der Trennplatte 71 ist damit diejenige Begrenzungsfläche der Trennplatte 71 gemeint, welche der Entlastungsöffnung 104 zugewandt ist. Jeder Befestigungssteg 72 erstreckt sich von der Unterseite der Trennplatte 71 in axialer Richtung A bis zu der Ummantelung 102, auf welcher sich der Befestigungssteg 72 abstützt. Die Trennplatte 71 ist somit mittels der Befestigungsstege 72 an der Ummantelung 102 fixiert. Daher ist es nicht mehr nötig, die Trennplatte 71 bzw. das Trennelement 7 an den Flügeln 103 zu befestigen.

Wie dies am besten in Fig. 5 zu erkennen ist, sind die Befestigungsstege 72 bezüglich der radialen Richtung auf einem Kreis angeordnet, der konzentrisch mit der Entlastungsöffnung 104 ist und einen grösseren Durchmesser ausweist als die Entlastungsöffnung 104. Der Durchmesser des Kreises, auf welchem die Befestigungsstege angeordnet sind, ist kleiner als der Aussendurchmesser D2 der Trennplatte 71. Die Befestigungsstege 72 sind also derart an der Unterseite der Trennplatte 71 angeordnet, dass sie vom Einlass 21 des Pumpengehäuses 2 aus nicht sichtbar sind.

Die Befestigungsstege 72 sind vorzugsweise äquidistant um die Entlastungsöffnung 104 herum angeordnet. Es sind insgesamt fünf Befestigungsstege 72 vorgesehen. Zwischen zwei benachbarten Befestigungsstegen ist jeweils eine radiale Öffnung 73 vorgesehen, durch welche der Rezirkulationsfluss RF aus der Entlastungsöffnung 104 in radialer Richtung in Richtung des Auslasses 22 strömen kann.

Vorzugsweise sind die Befestigungsstege 72 so angeordnet, dass die radialen Öffnungen 73 in radialer Richtung mit den Zwischenräumen 74 zwischen den Eintrittskanten 109 zweier benachbarter Flügel 103 fluchten. Dies ist am besten in Fig. 5 zu erkennen. Somit kann der aus den radialen Öffnungen 73 herausströmende Rezirkulationsfluss RF ungehindert in die Zwischenräume 74 zwischen benachbarten Flügeln 103 einströmen. Hierdurch werden Wirbelbildungen im Rezirkulationsfluss RF zumindest stark reduziert. Für diese Ausgestaltung ist es besonders vorteilhaft, wenn die Anzahl der Befestigungsstege 72 gleich der Anzahl der Flügel ist. Bei der hier beschriebenen Ausgestaltung hat der Rotor 10 mit beispielhaftem Charakter genau fünf Flügel. Dementsprechend hat das Trennelement 7 genau fünf Befestigungsstege 72, von denen jeder auf einer Verbindungslinie zwischen der Mittelachse M des Rotors 10 und einer der Eintrittskanten 109 der Flügel 103. Somit fluchtet jede der fünf radialen Öffnungen 73 in radialer Richtung jeweils mit genau einem der Zwischenräume 74 zwischen benachbarten Flügeln 103.

Erfindungsgemäss sind die Mehrzahl von Flügeln 103, das Trennelement 7 und die Entlastungsöffnung 104 als einstückige Einheit ausgestaltet. Besonders bevorzugt ist auch die Ummantelung 102 ein Bestandteil dieser einstückigen Einheit. Ferner ist es bevorzugt, dass auch die Deckplatte 8, die auf den Flügeln 103 angeordnet ist, Bestandteil dieser einstückigen Einheit ist. Besonders bevorzugt ist der Rotor 10 gesamthaft mit Ausnahme des magnetisch wirksamen Kerns 101 als eine einstückige Einheit ausgestaltet. Bei dieser Ausgestaltung ist es also nicht mehr notwendig, die einzelnen Komponenten des Rotor 10 durch Fügeverfahren wie Kleben, Schweissen, Verschrauben oder ähnliches miteinander zu verbinden. Die einstückige Einheit hat eine monolithische Ausgestaltung, sie ist also nicht aus mehreren Komponenten zusammengesetzt, sondern ein einziges Stück. Folglich ist die einstückige Einheit frei von Verklebungen, Verschraubungen, Schweissnähten, Dichtungen und Kontakten zwischen benachbarten Komponenten.

Durch die einstückige Ausgestaltung der Einheit, die mindestens die Flügel 103, das Trennelement 7 und die mindestens eine Entlastungsöffnung 104 umfasst, und vorzugsweise alle Komponenten des Rotors 10 mit Ausnahme des magnetisch wirksamen Kerns 101, muss der Rotor 10 nicht mehr aus mehreren Komponenten zusammengebaut werden, sondern kann als monolithische Vorrichtung ausgestaltet werden.

Da es daher keiner Verbindungen einzelner Komponenten durch beispielsweise Kleben, Verschrauben oder Schweissen sowie keiner Dichtungen zwischen einzelnen Komponenten des Rotors 10 bedarf, resultiert eine sehr hohe Betriebssicherheit.

Die einstückige Einheit ist beispielsweise als einstückiges Spritzgussteil ausgestaltet, kann also mittels eines Spritzgiessverfahren hergestellt. Das Spritzgiessverfahren ist dabei bevorzugt so ausgestaltet, dass der magnetisch wirksame Kern 101 in das Spritzgiessverfahren eingebunden ist. Der magnetisch wirksame Kern 101 kann beispielsweise in dem Spritzgiessverfahren mit einem Kunststoff umspritzt werden, um dadurch die Ummantelung 102 herzustellen.

Vorzugsweise wird der Rotor 10 durch die Kombination eines Spritzgiessverfahren mit einer anschliessenden subtraktiven Bearbeitungsmethode, beispielsweise einer spanabhebenden Bearbeitungsmethode wie Fräsen oder Bohren, hergestellt.

Natürlich sind auch andere Verfahren zur Herstellung des Rotors 10 geeignet, beispielsweise Verfahren der additiven Fertigung (additive manufacturing) wie z.B. das als 3D-Drucken bezeichnete Verfahren.

Vorzugsweise besteht die einstückige Einheit aus einem Kunststoff. Beispielsweise kann die einstückige Einheit aus einem der folgenden Kunststoffe spritzgegossen werden:
Polyvinylchlorid (PVC), Perfluoralkoxy-Polymere (PFA), Polypropylen (PP), Polyethylen (PE).

Auch ist es möglich, den Rotor 10 mittels eines Sinterprozesses und einer anschliessenden subtraktiven Bearbeitungsmethode herzustellen. Die Ummantelung 102 wird dann beispielsweise aus einem Pulver oder aus einem Granulat hergestellt, das unter Anwendung von Druck und optional eine Temperaturbehandlung auf den magnetisch wirksamen Kern 101 gepresst wird, derart, dass der magnetisch wirksame Kern 101 vollständig umschlossen wird. Mit Hitze und/oder Druck wird der Kunststoff um den magnetisch wirksamen Kern 101 herum zu einem monolithischen Block, beispielsweise zu einem zylinderförmigen Körper, geformt. Der Rotor 10 mit den Flügeln 103, dem Trennelement 7, der mindestens einen Entlastungsöffnung 104 und optional der Deckplatte 8 wird dann durch eine spanabhebenden Bearbeitung in die gewünschte Form gebracht.

Ferner ist es möglich, zusätzlich oder anstatt eines Pulvers oder eines Granulats mehrere Kunststoffteile mit Hitze und/oder Druck zu einem monolithischen Block zu fügen, in welche der Magnet vorab eingelegt wurde und welche den Magnet nach dem Fügeprozess vollständig umschliessen. Der Rotor 10 mit den Flügeln 103, dem Trennelement 7, der mindestens einen Entlastungsöffnung 104 und optional der Deckplatte 8 wird dann durch eine spanabhebenden Bearbeitung in die gewünschte Form gebracht.

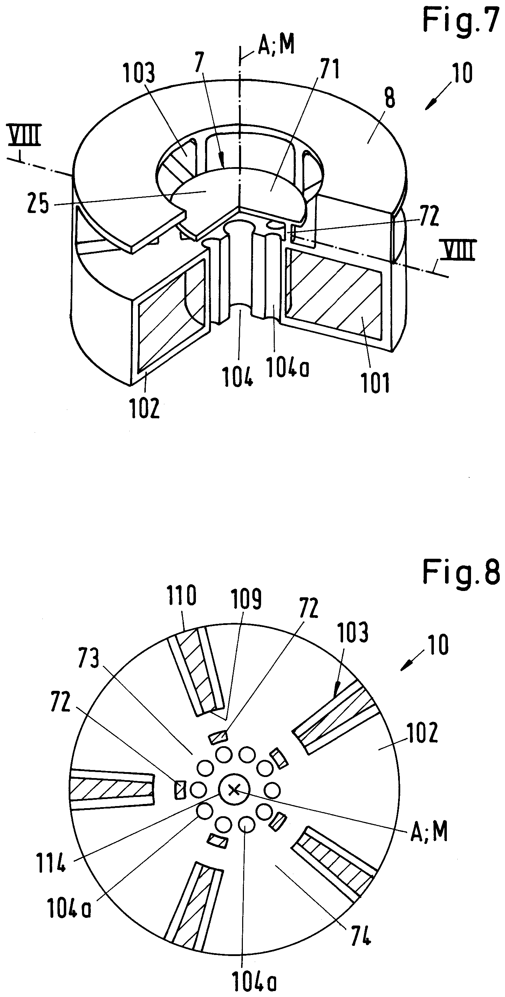

In den Fig. 6 - Fig. 8 ist eine erste Variante des Rotors 10 dargestellt. Fig. 6 zeigt den Rotor in einer Schnittdarstellung, wobei der Schnitt entlang der axialen Richtung A erfolgt. Fig. 7 zeigt eine perspektivische Darstellung, teilweise im Schnitt, welche der Darstellung in Fig. 4 entspricht. Fig. 8 zeigt eine Schnittdarstellung der ersten Variante des Rotors 10 entlang der Schnittlinie VIII-VIII in Fig. 7. Die Darstellung in Fig. 8 entspricht derjenigen in Fig. 5.

Bei der ersten Variante des Rotors 10 sind mehrere Entlastungsöffnungen 104, 104a vorgesehen. Eine der Entlastungsöffnungen 104 ist wiederum zentral im Rotor 10 angeordnet, sodass die Achse dieser Entlastungsöffnung 104 mit der Mittelachse M des Rotors 10 zusammenfällt. Um diese zentral angeordnete Entlastungsöffnung 104 herum ist eine Vielzahl von zusätzlichen Entlastungsöffnungen 104a angeordnet. Mit beispielhaftem Charakter sind hier zehn zusätzliche Entlastungsöffnungen 104a vorgesehen, die auf einem Kreis angeordnet sind, dessen Mittelpunkt auf der Mittelachse des Rotors 10 liegt. Jede der Entlastungsöffnungen 104, 104a ist jeweils als zylindrische Bohrung oder Öffnung ausgestaltet welche sich von dem zentralen Einlassbereich 25 in axialer Richtung A durch den Rotor 10 bis zu seiner Rückseite erstreckt. Alle Entlastungsöffnungen 104, 104a sind parallel zueinander angeordnet Der Kreis, auf dem die zusätzlichen Entlastungsöffnungen 104a angeordnet sind, hat einen Durchmesser, der kleiner ist als der Aussendurchmesser D2 der Trennplatte 71, derart, dass alle Entlastungsöffnungen 104, 104a von der Trennplatte 71 vollständig überdeckt werden. Somit ist keine der Entlastungsöffnungen 104, 104a vom Einlass 21 aus sichtbar.

Es sind aber auch solche Ausgestaltungen möglich, bei welchen eine oder mehrere der Entlastungsöffnungen 104, 104a teilweise oder vollständig vom Einlass 21 aus sichtbar sind, bei welchen also die Trennplatte 71 nicht alle Entlastungsöffnungen 104, 104a vollständig überdeckt. Dies kann beispielsweise so realisiert werden, dass der Aussendurchmesser D2 der Trennplatte 71 gleich gross oder kleiner ist als der Durchmesser des Kreises, auf welchem die zusätzlichen Entlastungsöffnungen 104a angeordnet sind.

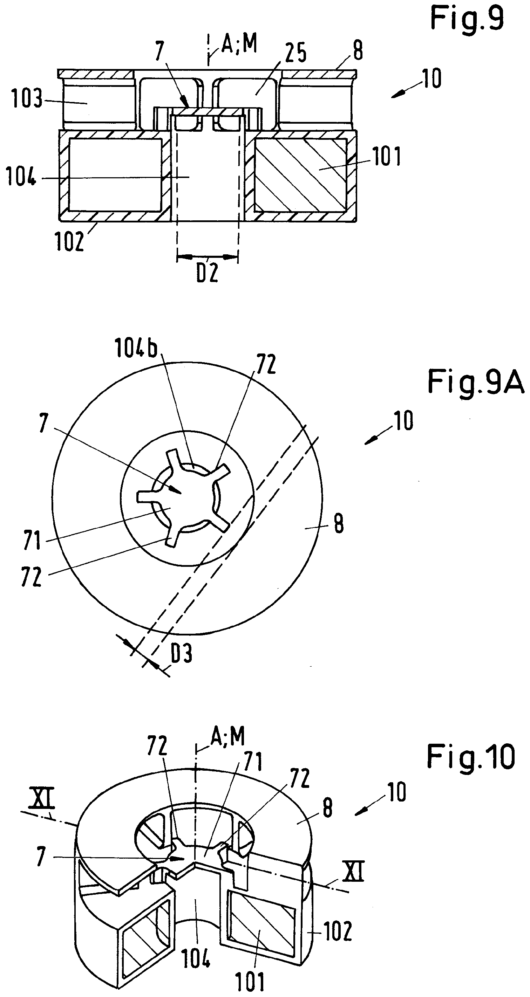

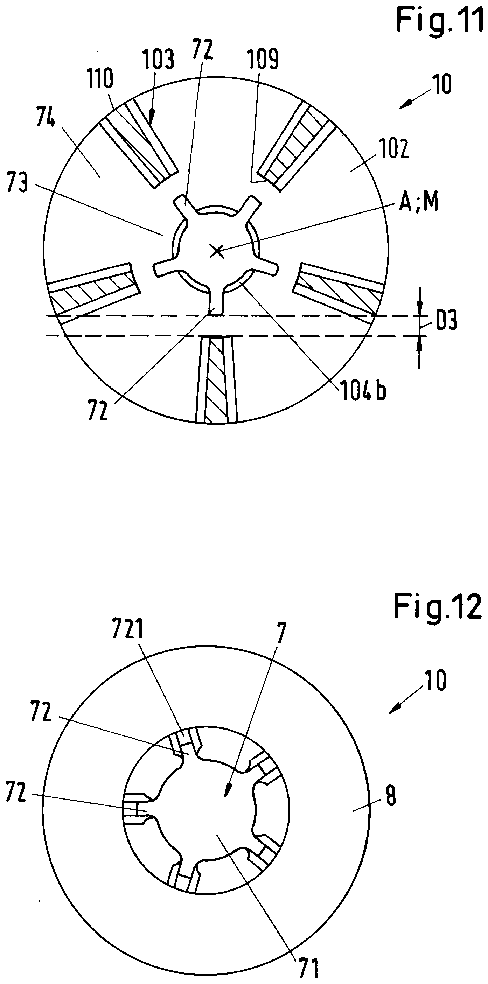

In den Fig. 9, Fig. 9A, Fig. 10 und Fig. 11 ist eine zweite Variante des Rotors 10 dargestellt. Fig. 9 zeigt den Rotor 10 in einer Schnittdarstellung, wobei der Schnitt entlang der axialen Richtung A erfolgt. Fig. 9A zeigt den Rotor 10 in einer Aufsicht vom Einlass 21 des Pumpengehäuses 2. Fig. 10 zeigt eine perspektivische Darstellung, teilweise im Schnitt, welche der Darstellung in Fig. 4 entspricht. Fig. 11 zeigt eine Schnittdarstellung der zweiten Variante des Rotors 10 entlang der Schnittlinie XI-XI in Fig. 10. Die Darstellung in Fig. 11 entspricht derjenigen in Fig. 5.

Bei der zweiten Variante des Rotors 10 ist wiederum nur eine Entlastungsöffnung 104 vorgesehen, welche zentral angeordnet ist. Es versteht sich, dass auch solche Ausgestaltungen der zweiten Variante des Rotors 10 möglich sind, bei welcher mehrere Entlastungsöffnungen 104, 104a vorgesehen sind, beispielsweise in sinngemäss gleicher Weise wie das für die erste Variante des Rotors 10 beschrieben ist (siehe Fig. 8).

Bei der zweiten Variante des Rotors 10 sind die Befestigungsstege 71 des Trennelement 7, mit denen die Trennplatte 71 fixiert wird, am Aussenrand der Trennplatte 71 angeordnet. Jeder Befestigungssteg 72 erstreckt sich vom Aussenrand der Trennplatte 7 in radialer Richtung nach aussen. Zudem erstreckt sich jeder Befestigungssteg 71 auch in axialer Richtung A bis auf die Ummantelung 102, auf welcher sich der Befestigungssteg 71 abstützt. Auch bei dieser Ausgestaltung mit der am Aussenrand der Trennplatte 71 angeordneten Befestigungsstegen 72 ist es bevorzugt, dass die Anzahl der Befestigungsstege 72, hier beispielsweise fünf, gleich gross ist wie die Anzahl der Flügel 103 des Rotors 10. Vom Einlass aus betrachtet, hat das Trennelement 7 mit der Trennplatte 71 und den an ihrem Rand angeordneten Befestigungsstegen 72 ein sternförmiges Aussehen.

Die Befestigungsstege 72 sind vorzugsweise äquidistant am Aussenrand der Trennplatte 71 angeordnet. Zwischen zwei benachbarten Befestigungsstegen 72 ist jeweils eine der radiale Öffnung 73 angeordnet, durch welche der Rezirkulationsfluss RF aus der Entlastungsöffnung 104 in radialer Richtung in Richtung des Auslasses 22 strömen kann.

Vorzugsweise sind die Befestigungsstege 72 auch bei der zweiten Variante des Rotors 10 so angeordnet, dass die radialen Öffnungen 73 in radialer Richtung mit den Zwischenräumen 74 zwischen den Eintrittskanten 109 zweier benachbarter Flügel 103 fluchten. Dies ist am besten in Fig. 11 zu erkennen. Somit kann der aus den radialen Öffnungen 73 herausströmende Rezirkulationsfluss RF ungehindert in die Zwischenräume 74 zwischen benachbarten Flügeln 103 einströmen. Hierdurch werden Wirbelbildungen im Rezirkulationsfluss RF zumindest stark reduziert.

Bei der zweiten Variante des Rotors 10 ist die Trennplatte 71 des Trennelements 10 wiederum in Form einer Kreisscheibe ausgestaltet. Wie dies in Fig. 9 zu erkennen ist, ist bei der zweiten Variante des Rotors 10 der Aussendurchmesser D2 der Trennplatte 71 kleiner als der Innendurchmesser der zentral angeordneten Entlastungsöffnung 104. Dadurch ist die Entlastungsöffnung 104 teilweise vom Einlass 21 aus sichtbar, denn die Trennplatte 71 überdeckt die Entlastungsöffnung 104 nicht vollständig. Um die Trennplatte 71 herum existiert als ein ringförmiger Spalt 104b, der von der Trennplatte 71 nicht überdeckt wird und vom Einlass 21 des Pumpengehäuses 2 aus sichtbar ist. Diese Ausgestaltung hat den Vorteil, dass bei der Herstellung des Rotors 10 ein Fräswerkzeug in axialer Richtung A in den ringförmigen Spalt 104 b eintauchen kann, wodurch sich insbesondere die Trennplatte 71 im Bereich zwischen zwei benachbarten Befestigungsstegen 72 einfacher oder genauer bearbeiten lässt.

Ferner ist es bevorzugt, dass die Befestigungsstege 72 bezüglich der radialen Richtung in einer Distanz D3 vor den Eintrittskanten 109 der Flügel 103 enden, wobei D3 ausreichend gross ist, sodass ein Fräswergzeug zwischen den Befestigungsstegen 72 und den Eintrittskanten 109 der Flügel 103 hindurchpasst. D3 ist also der in radialer Richtung gemessenen Abstand zwischen dem radial äusseren Ende der Befestigungsstege 72 und den Eintrittskannten 109 der Flügel 103. Bei der in Fig. 11 dargestellten Ausgestaltung D3 ist die Distanz D3 gleich dem in radialer Richtung gemessenen Abstand zwischen den radial äusseren Enden der Befestigungsstege 72 und der radial innenliegenden Kante der Deckplatte 8. Unter praktischen Aspekten ist es bevorzugt, dass die Distanz D3 mindestens so gross ist wie ein Dreissigstel, vorzugsweise mindestens so gross wie ein Fünfzehntel des in axialer Richtung A gemessene Abstand zwischen der Oberseite der Ummantelung 102, auf welcher die Flügel 103 angeordnet sind, und der dem Einlass 21 zugewandten und Oberseite des Rotors 10 am Einlassbereich 25, welche hier durch die Deckplatte 8 gebildet wird. Natürlich sind auch bei der zweiten Variante des Rotors solche Ausgestaltungen möglich, bei denen die Entlastungsöffnung 104 von der Trennplatte 71 vollständig überdeckt wird, sodass die Entlastungsöffnung 104 vom Einlass 21 aus nicht sichtbar ist. Dazu ist beispielsweise der Aussendurchmesser D2 der Trennplatte 71 grösser als der Innendurchmesser der Entlastungsöffnung 104.

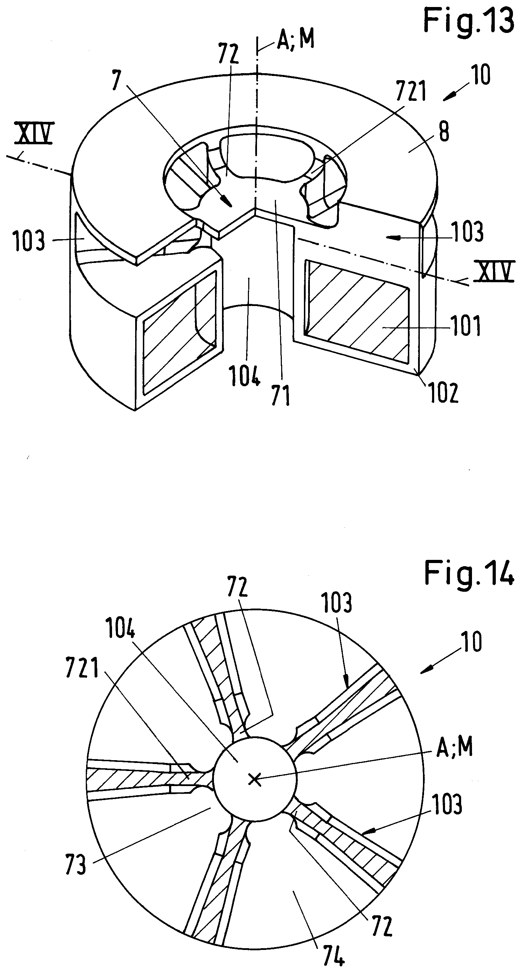

In den Fig. 12 - Fig. 14 ist eine dritte Variante des Rotors 10 dargestellt. Fig. 12 zeigt den Rotor 10 in einer Aufsicht vom Einlass des Pumpengehäuses 2. Fig. 13 zeigt eine perspektivische Darstellung, teilweise im Schnitt, welche der Darstellung in Fig. 4 entspricht. Fig. 14 zeigt eine Schnittdarstellung der dritten Variante des Rotors 10 entlang der Schnittlinie XIV-XIV in Fig. 13. Die Darstellung in Fig. 14 entspricht derjenigen in Fig. 5.

Die dritte Variante des Rotors 10 ist in ähnlicher Weise ausgestaltet wie die zweite Variante und insbesondere mit den Befestigungsstegen 72, die am Aussenrand der Trennplatte 71 angeordnet sind. Allerdings erstrecken sich die Befestigungsstege 72 in radialer Richtung bis an die Flügel 103 heran. Jeder Befestigungssteg 72 erstreckt sich also jeweils in radialer Richtung bis an die Eintrittskante 109 eines der Flügel 103. Vorzugsweise geht jeder der Befestigungsstege 72 jeweils in einen der Flügel 103 über. Bevorzugt ist am radial äusseren Ende jedes Befestigungsstegs 72 ein gerundet ausgestalteter Übergangsbereich 721 vorgesehen, in welchem der Befestigungssteg 72 in die Eintrittskante 109 des Flügels 103 übergeht.

Durch die Erfindung wird ferner die Zentrifugalpumpe 200 zum Fördern eines Fluids mit einer Pumpeneinheit 1 vorgeschlagen, wobei die Pumpeneinheit 1 erfindungsgemäss ausgestaltet ist. Fig. 15 zeigt in einer schematischen Schnittdarstellung ein Ausführungsbeispiel einer erfindungsgemässen Zentrifugalpumpe 200. Die Zentrifugalpumpe 200 umfasst den Stator 100, der sich in der axialen Richtung A von einem ersten axialen Ende 110 bis zu einem zweiten axialen Ende 120 erstreckt, wobei an dem ersten axialen Ende 110 eine becherförmige Ausnehmung (in Fig. 15 nicht dargestellt) vorgesehen ist, in welche der zylindrischen Becher 31 der Pumpeneinheit 1 einsetzbar ist. Der Stator 100 bildet mit dem Rotor 10 einen elektromagnetischen Drehantrieb zum Rotieren des Rotors 10 um die axiale Richtung A, wobei der Stator 100 als Lager- und Antriebsstator ausgestaltet ist, mit welchem der Rotor 10 berührungslos magnetisch antreibbar und berührungslos magnetisch bezüglich des Stators 100 lagerbar ist, wobei der Rotor 10 bezüglich der axialen Richtung A passiv magnetisch stabilisiert ist, und in einer zur axialen Richtung A senkrechten radialen Ebene E aktiv magnetisch gelagert ist.

Der Stator 100 umfasst ein Statorgehäuse, das in Fig. 15 aus Gründen der besseren Übersicht nicht dargestellt ist. Der Stator 100 kann aber beispielsweise in analoger Weise ausgestaltet sein, wie der in Fig. 1 dargestellte Stator 100' mit dem Statorgehäuse 130', wobei in dem Statorgehäuse 130' die Ausnehmung 121' vorgesehen ist, in welche der zylindrische Becher 31 des Bodenteils 3 des Pumpengehäuses 1 eingesetzt wird.

Besonders bevorzugt ist der elektromagnetische Drehantrieb mit dem Rotor 10 und dem Stator 100 als Tempelmotor ausgestaltet, wobei der Stator 100 eine Mehrzahl von Spulenkernen 125 aufweist, von denen jeder einen Längsschenkel 126 umfasst, welcher sich von einem ersten Ende in axialer Richtung A bis zu einem zweiten Ende erstreckt, sowie einen Querschenkel 127, welcher an dem zweiten Ende des Längsschenkels 126 und in der radialen Ebene E angeordnet ist. Der Querschenkel 127 erstreckt sich von dem Längsschenkel 126 in radialer Richtung nach innen auf den Rotor 10 zu.

Alle ersten Enden der Längsschenkel 126 - also die darstellungsgemäss unteren Enden - sind durch einen Rückschluss 122 zum Führen des magnetischen Flusses miteinander verbunden.

Die Spulenkerne 125 sind bezüglich der Umfangsrichtung um den Rotor 10 herum angeordnet sind, sodass der Rotor 10 zwischen den Querschenkeln 127 der Spulenkerne 125 angeordnet ist. An jedem Längsschenkel 126 ist mindestens eine konzentrierte Wicklung 160 vorgesehen, welche den jeweiligen Längsschenkel 126 umgibt.

Mit den konzentrierten Wicklungen 160 werden die für den magnetischen Antrieb und die magnetische Lagerung des Rotors 10 notwendigen elektromagnetischen Felder erzeugt. Mit diesen konzentrierten Wicklungen 160 werden im Betriebszustand also diejenigen elektromagnetischen Felder erzeugt, mit welchen in an sich bekannter Weise ein Drehmoment auf den Rotor 10 bewirkt wird, und mit welchen eine beliebig einstellbare Querkraft in radialer Richtung auf den Rotor 10 ausübbar ist, sodass die radiale Position des Rotors 10, also seine Position in der zur axialen Richtung A senkrechten radialen Ebene E, aktiv steuerbar bzw. regelbar ist. Bezüglich dreier weiterer Freiheitsgrade, nämlich seiner Position in axialer Richtung A und Verkippungen bezüglich der zur Solldrehachse senkrechten radialen Ebene E (zwei Freiheitsgrade), ist der Rotor 10 passiv magnetisch, das heisst nicht ansteuerbar, durch Reluktanzkräfte gelagert bzw. stabilisiert.

## Patentansprüche

1. Pumpeneinheit für eine Zentrifugalpumpe, welche die Pumpeneinheit und einen Stator (100) umfasst, der sich in einer axialen Richtung (A) von einem ersten axialen Ende (110) bis zu einem zweiten axialen Ende (120) erstreckt, wobei an dem ersten axialen Ende (110) eine becherförmige Ausnehmung vorgesehen ist, in welche die Pumpeneinheit (1) einsetzbar ist, wobei die Pumpeneinheit (1) ein Pumpengehäuse (2) mit einem Einlass (21) und mit einem Auslass (22) für ein zu förderndes Fluid aufweist, sowie einen in dem Pumpengehäuse (2) angeordneten Rotor (10) mit einer Mehrzahl von Flügeln (103) zum Fördern des Fluids, wobei der Rotor (10) um die axiale Richtung (A) rotierbar ist, wobei die Pumpeneinheit (1) für eine berührungslos magnetische Lagerung des Rotors (10) und für einen berührungslos magnetischen Antrieb des Rotors (10) durch den Stator (100) ausgestaltet ist, wobei die Flügel (103) des Rotors (10) um einen zentralen Einlassbereich (25) des Rotors (10) herum angeordnet sind, wobei der Rotor (10) mindestens eine Entlastungsöffnung (104, 104a) zur Erzeugung eines Rezirkulationsflusses (RF) umfasst, der von einer dem Einlass (21) abgewandten Rückseite des Rotors (10) in Richtung des zentralen Einlassbereichs (25) des Rotors (10) gerichtet ist, und wobei der Rotor (10) ferner ein im zentralen Einlassbereich (25) angeordnetes Trennelement (7) aufweist, welches den Rezirkulationsfluss (RF) in eine zur axialen Richtung (A) senkrechte radiale Richtung umlenkt, **dadurch gekennzeichnet, dass** die Mehrzahl von Flügeln (103), das Trennelement (7) und die mindestens eine Entlastungsöffnung (104, 104a) des Rotors (10) als einstückige Einheit ausgestaltet sind.

2. Pumpeneinheit nach Anspruch 1, wobei genau eine Entlastungsöffnung (104) vorgesehen ist, welche den zentralen Einlassbereich (25) des Rotors mit der Rückseite des Rotors (10) verbindet.

3. Pumpeneinheit nach Anspruch 1, wobei mehrere Entlastungsöffnungen (104, 104a) vorgesehen sind, welche um eine Mittelachse (M) des Rotors herum angeordnet sind, und wobei jede Entlastungsöffnung (104, 104a) den zentralen Einlassbereich (25) des Rotors mit der Rückseite des Rotors (10) verbindet.

4. Pumpeneinheit nach einem der vorangehenden Ansprüche, wobei der Rotor (10) einen ringförmigen oder scheibenförmigen magnetisch wirksamen Kern (101) umfasst, sowie eine Ummantelung (102), welche den magnetisch wirksamen Kern (101) vollständig umschliesst, und wobei die Ummantelung (102) ein Bestandteil der einstückigen Einheit ist, welche die Flügel (103) und das Trennelement (7) umfasst.

5. Pumpeneinheit nach einem der vorangehenden Ansprüche, wobei das Trennelement (7) derart ausgestaltet und angeordnet ist, dass die mindestens eine Entlastungsöffnung (104, 104a) vom Einlass aus teilweise sichtbar ist.

6. Pumpeneinheit nach einem der vorangehenden Ansprüche, wobei das Trennelement (7) eine Trennplatte (71) und Befestigungsstege (72) umfasst, wobei die Trennplatte (71) einen maximalen Aussendurchmesser (D2) in radialer Richtung aufweist, der höchstens so gross ist wie der Durchmesser (D1) des zentralen Einlassbereichs (25) des Rotors (10), und wobei die Befestigungsstege (72) zum Fixieren der Trennplatte (71) ausgestaltet sind.

7. Pumpeneinheit nach Anspruch 6, wobei der maximale Aussendurchmesser (D2) der Trennplatte (71) kleiner ist als der Durchmesser (D1) des zentralen Einlassbereichs (25) des Rotors (10).

8. Pumpeneinheit nach Anspruch 4 und einem der Ansprüche 6-7, wobei sich jeder Befestigungssteg (72) von der Trennplatte (7) bis zu der Ummantelung (102) erstreckt, und wobei zwischen benachbarten Befestigungsstegen (72) jeweils eine radiale Öffnung (73) für den Rezirkulationsfluss (RF) vorgesehen ist.

9. Pumpeneinheit nach Anspruch 8, wobei sich jeder Befestigungssteg (72) von einer Unterseite der Trennplatte (71) in axialer Richtung (A) bis zu der Ummantelung (102) erstreckt.

10. Pumpeneinheit nach Anspruch 8, wobei jeder Befestigungssteg (72) am Aussenrand der Trennplatte (7) angeordnet ist, und sich vom Aussenrand in radialer Richtung erstreckt.

11. Pumpeneinheit nach Anspruch 10, wobei die Befestigungsstege (72) äquidistant am Aussenrand der Trennplatte (71) angeordnet sind.

12. Pumpeneinheit nach einem der Ansprüche 10-11, wobei sich jeder Befestigungssteg (72) in radialer Richtung jeweils bis an einen der Flügel (103) erstreckt.

13. Pumpeneinheit nach einem der Ansprüche 10-12, wobei die Anzahl der Befestigungsstege (72) gleich der Anzahl der Flügel (103) ist.

14. Zentrifugalpumpe zum Fördern eines Fluids mit einer Pumpeneinheit, die nach einem der vorangehenden Ansprüche ausgestaltet ist, und die einen zylindrischen Becher (31) zur Aufnahme des Rotors (10) aufweist, sowie mit einem Stator (100), der sich in einer axialen Richtung (A) von einem ersten axialen Ende (110) bis zu einem zweiten axialen Ende (120) erstreckt, wobei an dem ersten axialen Ende (110) eine becherförmige Ausnehmung vorgesehen ist, in welche der zylindrischen Becher (31) der Pumpeneinheit (1) einsetzbar ist, wobei der Stator (100) mit dem Rotor (10) einen elektromagnetischen Drehantrieb zum Rotieren des Rotors (10) um die axiale Richtung (A) bildet, wobei der Stator (100) als Lager- und Antriebsstator ausgestaltet ist, mit welchem der Rotor (10) berührungslos magnetisch antreibbar und berührungslos magnetisch bezüglich des Stators (100) lagerbar ist, wobei der Rotor (10) bezüglich der axialen Richtung (A) passiv magnetisch stabilisiert ist, und in einer zur axialen Richtung (A) senkrechten radialen Ebene (E) aktiv magnetisch gelagert ist.

15. Zentrifugalpumpe nach Anspruch 14, bei welchem der elektromagnetische Drehantrieb als Tempelmotor ausgestaltet ist, wobei der Stator (100) eine Mehrzahl von Spulenkernen (125) aufweist, von denen jeder einen Längsschenkel (126) umfasst, welcher sich von einem ersten Ende in axialer Richtung (A) bis zu einem zweiten Ende erstreckt, sowie einen Querschenkel (127), welcher an dem zweiten Ende des Längsschenkels (126) und in der radialen Ebene (E) angeordnet ist, und welcher sich von dem Längsschenkel (126) in radialer Richtung erstreckt, wobei die Spulenkerne (125) bezüglich der Umfangsrichtung um den Rotor (10) herum angeordnet sind, sodass der Rotor (10) zwischen den Querschenkeln (127) der Spulenkerne (125) angeordnet ist, und wobei an jedem Längsschenkel (125) mindestens eine konzentrierte Wicklung (160) vorgesehen ist, welche den jeweiligen Längsschenkel (126) umgibt.
